# EUROPEAN PATENT SPECIFICATION

(11) **EP 3 429 580 B1**
(45) Date of publication and mention of the grant of the patent: **07.05.2025**
(21) Application number: 17765961.2
(22) Date of filing: 15.03.2017
(51) Int. Cl.: A61K 31/352, A61K 31/01, A61K 47/10, A61K 47/38

(54) **TERPENE-ENRICHED CANNABINOID COMPOSITION**
TERPENANGEREICHERTE CANNABINOIDZUSAMMENSETZUNG
COMPOSITION DE CANNABINOÏDES ENRICHIE EN TERPÈNE

(30) Priority: 16.03.2016 US 201662308961 P
(43) Date of publication of application: 23.01.2019
(60) Divisional application: 25165017.2
(73) Proprietor: Buzzelet Development And Technologies Ltd, 4065101 Or-Akiva (IL)
(72) Inventor: EYAL, Aharon M., 93629 Jerusalem (IL); RAZ, Noa, 9974500 Gizo (IL)
(74) Representative: Harrison IP Limited
(86) International application number: PCT/IB2017/051515
(87) International publication number: WO 2017/158539

(56) References cited:
- WO-A1-2014/100231
- WO-A1-2014/159688
- WO-A1-2015/065544
- WO-A1-2015/161165
- WO-A1-2016/030369
- WO-A1-2016/138505
- WO-A2-2015/068052
- WO-A2-2015/068052
- US-A1- 2008 112 895
- US-A1- 2014 243 405
- US-A1- 2014 271 940
- US-A1- 2016 039 591
- MEDIAVILLA VITO ET AL: "Essential oil of Cannabis sativa L. strains", JOURNAL OF THE INTERNATIONAL HEMP ASSOCIATION, vol. 4, no. 2, 1 January 1997 (1997-01-01), pages 80 - 82, XP093011851, Retrieved from the Internet <URL:https://www.druglibrary.net/olsen/HEMP/IHA/jiha4208.html>
- OIER AIZPURUA-OLAIZOLA ET AL: "Evolution of the Cannabinoid and Terpene Content during the Growth of Cannabis sativa Plants from Different Chemotypes", JOURNAL OF NATURAL PRODUCTS, vol. 79, no. 2, 26 February 2016 (2016-02-26), US, pages 324 - 331, XP055381197, ISSN: 0163-3864, DOI: 10.1021/acs.jnatprod.5b00949
- AIZPURUA-OLAIZOLA OIER: "Evolution of the Cannabinoid and Terpene Content during the Growth of Cannabis sativa Plants from Different Chemotypes", JOURNAL OF NATURAL PRODUCTS, vol. 79, no. 2, 26 February 2016 (2016-02-26), pages 327, XP093011867, Retrieved from the Internet <URL:doi:10.1021/acs.jnatprod.5b00949>
- RUSSO, TAMING THC: "Potential Cannabis Synergy and Phytocannabinoid-Terpenoid Entourage Effects", BRITISH JOURNAL OF PHARMACOLOGY, vol. 163, 2011, pages 1344 - 1364, XP055420723, Retrieved from the Internet <URL:https://www.ncbi.nlm.nih.gov/pmc/articles/PMC3165946/pdf/bph0163-1344.pdf> [retrieved on 20170523]

## Description

### Technical Field

The field of art to which this invention generally pertains is cannabinoid compositions, and specifically cannabinoid compositions for therapeutic use.

### Background

Terpenes play important roles in cannabinoids-comprising products, affecting the functionality and bioavailability of the cannabinoids and the aroma of the product. Processing cannabis plant material typically leads to terpenes loss so that most of cannabis products are of relatively low terpene content. That is particularly true for cannabis extracts and products thereof, such as cannabis tablets, cannabis gel capsules, cannabis patches, cannabis suppositories, etc. Most cannabis terpenes boiling points are in the range between about 150°C and about 220°C and they evaporate, at least partially, during cannabis buds drying, during solvent separation from extracts and during decarboxylation. Monoterpenes are lost at a rate greater than that of terpenes with a higher molecular weight and terpenes carrying no hydroxyl groups are lost at a rate greater than that of terpenes that do carry hydroxyl groups.

The international patent WO-2016030369 discloses a passive inhaler device comprising cannabidiol, or a variant or a derivative thereof (like THC, CBD), and a carrier, wherein the carrier is a vegetable oil and/ or any one of a terpene or a terpenoid, such as any one of a mono-, di-, or a tri-terpene or terpenoid. The terpenoid is one or more selected from the list of total terpene extract from cannabis sativa, 1,8- cineole, d-limonene, β-myrcene, α-pinene, linalool, β-caryophyllene, caryophyllene oxide, nerolidol, phytol, borneol. The composition as being provided in a form that is suitable for oral delivery as mist. The terpene-enriched cannabinoid composition show enhanced therapeutic synergistic effects. Used to treat cancer, psychosis, anxiety, depression, prevention or alleviation of pain, neuropatic pain. Example 6 shows the composition used 8,6 mg CBD and 50:50 mixture of peppermint oil and total cannabis sativa terpene extract to a total of about 75 mg.

### Summary of the Invention

Provided is a composition in the form of an extract or a product thereof, said composition comprising
(i) at least one cannabinoid;
(ii) a total primary terpene content forming at least 40% by weight of the total amount of terpenes in the composition;
(iii) at least 5% by weight of a non-cannabinoid, non-terpene carrier, wherein said carrier comprises less than 5% cellulose by weight;
(iv) less than 5% by weight glycol; and
(v) less than 5% by weight water;
for use in treating a condition selected from the group consisting of pain, anxiety, depression, a sleep disorder, autism and autism spectrum disorder (ASD) by oral administration,
wherein
a) when said condition is pain, said at least one cannabinoid comprises THC and/or CBD; said primary terpene is selected from the group consisting of beta-myrcene, linalool, beta-caryophyllene, humulene, eucalyptol, beta-amyrin and combinations thereof; and a total terpenes to total cannabinoids weight/weight ratio in said composition is between 0.05:1 and 0.5:1;
b) when said condition is depression, said at least one cannabinoid comprises CBD; said primary terpene is selected from the group consisting of limonene, linalool, myrcene, beta-caryophyllene and combinations thereof; and a total terpenes to total cannabinoids weight/weight ratio in said composition is between 0.1:1 and 0.5:1;
c) when said condition is anxiety, said at least one cannabinoid comprises CBD; said primary terpene is selected from the group consisting of limonene, linalool, beta-caryophyllene and combinations thereof and a total terpenes to total cannabinoids weight/weight ratio in said composition is between 0.1:1 and 0.5:1;
d) when said condition is ASD, said at least one cannabinoid comprises CBD; said primary terpene is selected from the group consisting of limonene, beta-caryophyllene, caryophyllene oxide, pinene, beta- myrcene, humulene, citronellol, eucalyptol and combinations thereof; and a total terpenes to total cannabinoids weight/weight ratio in said composition is between 0.05:1 and 0.5:1; and
e) when said condition is a sleep disorder, said at least one cannabinoid comprises CBD; said primary terpene is selected from the group consisting of linalool, beta-myrcene, beta-caryophyllene and combinations thereof; and a total terpenes to total cannabinoids weight/weight ratio in said composition is between 0.1:1 and 0.5:1.

According to some embodiments, the composition is for use in treating a condition selected from the group consisting of depression, anxiety, autism and ASD according to Claim 1, wherein said at least one cannabinoid further comprises THC and/or THCa at CBD to THC and/or THCa weight/weight ratio greater than 1.

According to some embodiments, the condition is for use in treating pain, wherein the pain comprises acute and/or chronic pain.

According to some embodiments, the chronic pain comprises chronic pain from a condition selected from the group consisting of multiple sclerosis, Fibromyalgia, cancer and peripheral neuropathy.

According to some embodiments, at least 50% of the cannabinoid is in decarboxylated form.

According to some embodiments, the composition is administered by:
(i) administering to a patient for a first period of time a first terpene-enriched cannabis composition comprising a first cannabinoid at a first cannabinoid amount and a first primary terpene at a first primary terpene amount, followed by
(ii) administering to the patient for a second period of time a second terpene-enriched cannabis composition comprising the first cannabinoid amount and a second primary terpenes at a second primary terpene amount.

### Detailed Description

Unless indicated otherwise, percent is weight percent and ratio is weight/weight ratio. Unless indicated otherwise, weight ratio means the ratio between weight content, e.g. in an aqueous solution containing 20% solute and 80% water, the solute to water weight ratio is 20:80 or 1:4.

The particulars shown herein are by way of example and for purposes of illustrative discussion of the various embodiments of the present invention only and are presented in the cause of providing what is believed to be the most useful and readily understood description of the principles and conceptual aspects of the invention. In this regard, no attempt is made to show details of the invention in more detail than is necessary for a fundamental understanding of the invention, the description making apparent to those skilled in the art how the several forms of the invention may be embodied in practice.

The present invention will now be described by reference to more detailed embodiments. This invention may, however, be embodied in different forms and should not be construed as limited to the embodiments set forth herein. Rather, these embodiments are provided so that this disclosure will be thorough and complete, and will fully convey the scope of the invention to those skilled in the art.

Unless otherwise defined, all technical and scientific terms used herein have the same meaning as commonly understood by one of ordinary skill in the art to which this invention belongs. The terminology used in the description of the invention herein is for describing particular embodiments only and is not intended to be limiting of the invention. As used in the description of the invention and the appended claims, the singular forms "a," "an," and "the" are intended to include the plural forms as well, unless the context clearly indicates otherwise.

Unless otherwise indicated, all numbers expressing quantities of ingredients, reaction conditions, and so forth used in the specification and claims are to be understood as being modified in all instances by the term "about." Accordingly, unless indicated to the contrary, the numerical parameters set forth in the following specification and attached claims are approximations that may vary depending upon the desired properties sought to be obtained by the present invention. At the very least, and not as an attempt to limit the application of the doctrine of equivalents to the scope of the claims, each numerical parameter should be construed in light of the number of significant digits and ordinary rounding approaches.

Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in their respective testing measurements. Every numerical range given throughout this specification will include every narrower numerical range that falls within such broader numerical range, as if such narrower numerical ranges were all expressly written herein.

Additional advantages of the invention will be set forth in part in the description which follows, and in part will be obvious from the description, or may be learned by practice of the invention. It is to be understood that both the foregoing general description and the following detailed description are exemplary and explanatory only and are not restrictive of the invention, as claimed.

Provided is a composition in the form of an extract or a product thereof, said composition comprising
(i) at least one cannabinoid;
(ii) a total primary terpene content forming at least 40% by weight of the total amount of terpenes in the composition;
(iii) at least 5% by weight of a non-cannabinoid, non-terpene carrier, wherein said carrier comprises less than 5% cellulose by weight;
(iv) less than 5% by weight glycol; and
(v) less than 5% by weight water;
for use in treating a condition selected from the group consisting of pain, anxiety, depression, a sleep disorder, autism and autism spectrum disorder (ASD) by oral administration,
wherein
a) when said condition is pain, said at least one cannabinoid comprises THC and/or CBD; said primary terpene is selected from the group consisting of beta-myrcene, linalool, beta-caryophyllene, humulene, eucalyptol, beta-amyrin and combinations thereof; and a total terpenes to total cannabinoids weight/weight ratio in said composition is between 0.05:1 and 0.5:1;
b) when said condition is depression, said at least one cannabinoid comprises CBD; said primary terpene is selected from the group consisting of limonene, linalool, myrcene, beta-caryophyllene and combinations thereof; and a total terpenes to total cannabinoids weight/weight ratio in said composition is between 0.1:1 and 0.5:1;
c) when said condition is anxiety, said at least one cannabinoid comprises CBD; said primary terpene is selected from the group consisting of limonene, linalool, beta-caryophyllene and combinations thereof and a total terpenes to total cannabinoids weight/weight ratio in said composition is between 0.1:1 and 0.5:1;
d) when said condition is ASD, said at least one cannabinoid comprises CBD; said primary terpene is selected from the group consisting of limonene, beta-caryophyllene, caryophyllene oxide, pinene, beta- myrcene, humulene, citronellol, eucalyptol and combinations thereof; and a total terpenes to total cannabinoids weight/weight ratio in said composition is between 0.05:1 and 0.5:1; and
e) when said condition is a sleep disorder, said at least one cannabinoid comprises CBD; said primary terpene is selected from the group consisting of linalool, beta-myrcene, beta-caryophyllene and combinations thereof; and a total terpenes to total cannabinoids weight/weight ratio in said composition is between 0.1:1 and 0.5:1.

According to an embodiment, said composition comprises at least two cannabinoids, at least three, at least four or at least five. According to an embodiment, the content of each cannabinoid in said composition is at least 10 parts per million (ppm). As known in the art, cannabinoids have an acid form and a non-acid form (which is also referred to as decarboxylated form, since it can be generated by decarboxylating the acid form). The acid form is indicated herein by the letter (a) at the end of the cannabinoid acronym, e.g. tetrahydrocannabiniolic acid is indicated as THCa, while the decarboxylated form is THC. According to an embodiment, said cannabinoids are selected from the group consisting of tetrahydrocannabiniol in acid or decarboxylated form (THCa or THC, respectively), cannabidiol in acid or decarboxylated form (CBDa or CBD, respectively), cannabigerol in acid or decarboxylated form (CBGa or CBG, respectively), cannabichromene in acid or decarboxylated form (CBCa or CBC, respectively) tetrahydrocannabivarin in acid or decarboxylated form (THCVa or THCV, respectively), Cannabidivarin in acid or decarboxylated form (CBDVa or CBDV respectively) and cannabinol in acid or decarboxylated form (CBNa or CBN, respectively).

According to an embodiment, at least one of said cannabinoids is in acid form. According to an embodiment, at least one of said cannabinoid is at least partially in decarboxylated form. According to an embodiment, at least 50% of said cannabinoid is in decarboxylated form, at least 60%, at least 70%, at least 80% or at least 90%.

According to an embodiment, said composition comprises THC and/or THCa. According to an embodiment, said composition comprises CBD and/or CBDa. According to an embodiment, said composition comprises THC and/or THCa at a content of less than 1%, less than 0.8%, less than 0.6%, less than 0.4% or less than 0.2%. According to an embodiment, said composition comprises both CBD and/or CBDa and THC and/or THCa and the weight/weight ratio between CBD and/or CBDa and THC and/or THCa ((CBD + CBDa)/(THC + THCa)) is at least 10, at least 15, at least 20, at least 25 or at least 30.

According to an embodiment, said composition comprises at least 5% by weight carrier, at least 10%, at least 15%, at least 20%, at least 25%, at least 30%, at least 35% or at least 40% by weight. Any compound other than cannabinoids and terpenes is a suitable carrier. According to an embodiment, said carrier is selected from the group consisting of vegetable oils, e.g. coconut oil, olive oil or sesame oil, pharmaceutical excipients, honey, bees wax, cellulose at less than 5% by weight, and combinations thereof. As used herein, the term cellulose refers to cellulose, hemicellulose and their combinations.

According to an embodiment, said composition comprises less than 5% by weight glycol, less than 4%, less than 3%, less than 2%, or less than 1%, by weight glycol. As used herein, the term glycol refers to any glycol, including ethylene glycol, polyethylene glycol, propylene glycol and polypropylene glycol.

According to an embodiment, said composition comprises water and water content is less than 5% by weight. According to an embodiment, water content is at least 1% by weight, at least 2%, at least 3%, at least 4% or 5%.

According to an embodiment, said composition comprises a primary terpene and optionally at least three secondary terpenes, at least four or at least five. The term "terpene", as used herein, refers to both terpenes and terpenoids. As used here, the term "primary terpene" refers to a terpene that forms at least 20% by weight of the total amount of terpenes in the said composition, at least 30%, at least 40%, at least 50%, at least 60%, at least 70% or at least 80%. According to an embodiment, said composition comprises multiple (e.g. two or three) terpenes, each one of which forms at least 20% by weight of the total amount of terpenes in the said composition and each one of these terpenes is considered a primary terpene. As used here, the term "secondary terpene" refers to a terpene that forms at least 10 parts per million (ppm) of said composition. According to an embodiment, the content of said primary terpene in said composition is at least 2 times greater than that of any secondary terpene, at least 4, at least 6, at least 8, at least 10, at least 15, at least 20, at least 25, or at least 30 times greater.

As used herein, "terpenes/cannabinoids (or terpenes to cannabinoids) weight/weight ratio" means the weight ratio between the combined amount of terpenes and the combined amount of cannabinoids.

According to another embodiment, said non-cannabinoid, non-terpene carrier comprises less than 5% cellulose and terpenes to cannabinoids weight/weight ratio in said composition is in the range between about 0.05 and about 1.0. According to an embodiment, said ratio is greater than 0.1, greater than 0.15, greater than 0.2, greater than 0.25, greater than 0.3, greater than 0.35, greater than 0.4, greater than 0.45, greater than 0.5, greater than 0.55, greater than 0.6, greater than 0.65, greater than 0.7, greater than 0.75, greater than 0.8, greater than 0.85, greater than 0.9, greater than 0.95, greater than 1, greater than 1.2, greater than 1.5, greater than 2.0, greater than 3 or greater than 5. According to an embodiment, said ratio is less than 0.9, less than 0.8, less than 0.7, less than 0.6, less than 0.5, less than 0.4, less than 0.3, less than 0.2, less than 0.15 or less than 0.1. According to an embodiment, said composition comprising less than 5% cellulose is selected from the group consisting of cannabis trichomes, cannabis extracts and products thereof, such as tablets, gel capsules, medical patches, vaporizer liquids and suppositories.

According to an embodiment, at least one of said terpenes is a-cyclic. According to an embodiment, at least one of said terpenes is cyclic. According to an embodiment, at least one of said terpenes is not found in cannabis buds or is present there at less than 0.2%, less than 0.1%, less than 0.05% or less than 0.02%. Such terpene is referred to as "non-cannabis terpene". According to an embodiment, said terpene-enriched cannabinoid composition comprises said non-cannabis terpene at a concentration of at least 0.2%, at least 0.5%, least 0.8%, at least 1%, least 1.5%, at least 2%, at least 3%, least 4%, at least 5%, at least 8% or at least 12%.

According to an embodiment, at least one of said terpenes is a monoterpene, at least one of said terpenes is a sesquiterpene and the monoterpenes to sesquiterpenes weight/weight ratio (i.e. the weight ratio between the total amount of monoterpenes and the total amount of the sesquiterpenes) is greater than 1.5 greater than 2, greater than 2.5, greater than 3, greater than 3.5, greater than 4, greater than 4.5, greater than 5, greater than 6, greater than 7, greater than 8, greater than 9, greater than 10, greater than 15, or greater than 20.

According to an embodiment, at least one of said terpenes is a monoterpene, at least one of said terpenes is a diterpene and the monoterpenes to diterpenes weight/weight ratio (i.e. the weight ratio between the total amount of monoterpenes and the total amount of the diterpenes) is greater than 5, greater than 6, greater than 7, greater than 8, greater than 9, greater than 10, greater than 12, greater than 14, greater than 16, greater than 18, greater than 20, greater than 25, or greater than 30.

According to an embodiment, at least one of the terpenes carries no hydroxyl group, at least one of the terpenes carries hydroxyl group and the non-hydroxy-terpenes to hydroxyl-terpenes weight/weight ratio (i.e. the weight ratio between the total amount of non-hydroxy-terpenes and the total amount of the hydroxy-terpenes) is greater than 1.5, greater than 2, greater than 2.5, greater than 3, greater than 3.5, greater than 4, greater than 4.5, greater than 5, greater than 6, greater than 7, greater than 8, greater than 9, greater than 10, greater than 15, or greater than 20.

According to an embodiment, said composition is liquid while at 30°C. According to an embodiment, said composition is a suspension, while at 30°C. According to an embodiment, said composition is essentially clear of haze or suspended solids, while at 30°C.

According to an embodiment, said composition comprises cannabis plant material.

According to an embodiment, said composition comprises less than 5%wt cellulose and THC and/or THCa and/or CBD and/or CBDa in a concentration of at least 10% by weight, at least 15%, at least 20%, at least 25%, at least 30%, at least 35%, at least 40%, at least 45% or at least 50% by weight. According to an embodiment, said composition comprises less than 5%wt cellulose and water in a concentration of less than 5% by weight, less than 4%, less than 3%, less than 2%, or less than 1% by weight. According to an embodiment, said composition comprises less than 5% by weight cellulose, and said primary terpenes forms at least 40% by weight of the total terpene content, at least 50%, at least 60%, at least 70%, at least 80% or at least 90% by weight. According to an embodiment, said composition is liquid. According to an embodiment, said composition is selected from the group consisting of vaporizer cannabis filling liquid, cannabis tablets, cannabis suppositories, cannabis gel capsules, cannabis candies, cannabis drinks and cannabis baked products.

According to an embodiment, said composition comprises an additive selected from the group consisting of antioxidants, emulsifiers and texturizers vegetable oils, plant extracts, honey, pharmaceutical excipients, sucrose, glucose and fructose, pharmaceutical excipients and combinations thereof. According to an embodiment, said composition comprises a surfactant selected from the group consisting of phospholipids, glycerides, glycolipids and combinations thereof. According to an embodiment, said composition further comprises a food-approved texturizer. According to an embodiment, said composition further comprises at least 10ppm ethanol. According to an embodiment, said composition further comprises at least one of vitamin C, vitamin E, polyunsaturated fatty acids, beeswax and coconut oil. According to an embodiment, said product further comprises a sweetener.

According to an embodiment, said composition is selected from the group consisting of cannabis tablets, cannabis gel capsules, cannabis medical patches, cannabis cigarettes and cannabis vaporizer liquids, cannabis candies, cannabis drinks and cannabis baked products.

According to an embodiment, said composition results in an increased therapeutic effect compared with that of a composition comprising the same cannabinoids amounts and a smaller amount of said primary terpene, e.g. one half of that amount. According to various embodiment, said increased therapeutic effect has various forms, e.g. a shorter onset time, increased magnitude, extended duration, reduced dosages, reduced secondary adverse symptoms, and combinations thereof. According to an embodiment, said increased therapeutic effect comprises a shorter onset time, or differently put an earlier effect, which is important particularly in cases of sublingual and topical delivery and in cases where a rapid effect is desired, as in treating pain. According to an embodiment, said increased therapeutic effect comprises extended duration of the therapeutic effect, for example an extended time of pain relief. According to an embodiment, said increased therapeutic effect comprises increased magnitude of the therapeutic effect, enabling achieving a desired therapeutic effect on administering smaller doses of cannabinoids, saving thereby on cost. According to an embodiment, said increased therapeutic effect comprises using smaller doses of cannabinoids and still achieving at least the same beneficial result. According to an embodiment, said increased therapeutic effect comprises reduction of secondary adverse symptoms, e.g. adverse symptoms of the main illness, of ones of another illness and/or ones related to administered said composition or other drugs.

According to an embodiment, the primary terpene and the cannabinoids are present in specific amounts, and the onset time of said therapeutic effect, as measured by methods known in the art, is at least 20% shorter than that of a composition comprising the same cannabinoids amounts and one half the amount of said primary terpene, at least 30%, at least 40%, at least 50% or at least 60% shorter. According to an embodiment, the primary terpene, secondary terpenes and the cannabinoids are present in specific amounts, and the onset time of said therapeutic effect is at least 20% shorter than that of a composition comprising the same cannabinoids amounts, same secondary terpene amounts and one half the amount of said primary terpene, at least 30%, at least 40%, at least 50% or at least 60% shorter. Shorter onset time, or differently put an earlier effect, is important particularly in cases of sublingual and topical delivery and in cases where a rapid effect is desired, as in treating pain.

According to an embodiment, the primary terpene and the cannabinoids are present in specific amounts, and the onset time of said therapeutic effect is at least 20% longer than that of a composition comprising the same cannabinoids amounts and one half the amount of said primary terpene, at least 30%, at least 40%, at least 50% or at least 60% longer. According to an embodiment, the primary terpene, secondary terpenes and the cannabinoids are present in specific amounts, and the onset time of said therapeutic effect is at least 20% longer than that of a composition comprising the same cannabinoids amounts, same secondary terpene amounts and one half the amount of said primary terpene, at least 30%, at least 40%, at least 50% or at least 60% longer. According to an embodiment, compositions of delayed onset time are used in combination with shorter onset time to reach a sustained release effect.

According to an embodiment, the primary terpene and the cannabinoids are present in specific amounts, and the magnitude of the therapeutic effect, as measured by methods known in the art, is at least 20% greater compared with that of a composition comprising the same cannabinoids amounts and one half the amount of said primary terpene, at least 30%, at least 40%, at least 50% or at least 60% greater. According to an embodiment, the primary terpene, secondary terpenes and the cannabinoids are present in specific amounts, and the magnitude of the therapeutic effect is at least 20% greater than that of a composition comprising the same cannabinoids amounts, same secondary terpene amounts and one half the amount of said primary terpene, at least 30%, at least 40%, at least 50% or at least 60% greater. Without wishing to be limited by any particular theory, such increased magnitude of the therapeutic effect may indicate increased bioavailability. Such increased magnitude enables achieving a desired therapeutic effect on administering smaller doses of cannabinoids, saving thereby on cost.

According to an embodiment, the primary terpene and the cannabinoids are present in specific amounts, and the duration of the therapeutic effect, as measured by methods known in the art, is at least 20% longer compared with that of a composition comprising the same cannabinoids amounts and one half the amount of said primary terpene, at least 30%, at least 40%, at least 50% at least 60%.at least 70%, at least 80%, at least 90% or at least 100% longer. According to an embodiment, the primary terpene, secondary terpenes and the cannabinoids are present in specific amounts, and the duration of the therapeutic effect is at least 20% longer than that of a composition comprising the same cannabinoids amounts, same secondary terpene amounts and one half the amount of said primary terpene, at least 30%, at least 40%, at least 50%, at least 60%.at least 70%, at least 80%, at least 90% or at least 100% longer.

According to an embodiment, said enhanced therapeutic effect is observed in treating at least one condition selected from the group consisting of anxiety, depression, autism, autism spectrum disorder, pain, including pain from multiple sclerosis, Parkinson disease and Alzheimer's diseaseand sleep disorders.

According to an embodiment, said therapeutic effect is generated while administering said composition in a vaporizer. According to an embodiment, said therapeutic effect is generated while administering said composition sublingually.

According to an embodiment, said composition comprises CBD and optionally THC and/or THCa at CBD to THC and/or THCa weight/weight ratio greater than 1, greater than 2, greater than 3, greater than 4 or greater than 5, and said primary terpene is selected from the group consisting of limonene, linalool, and beta-caryophyllene resulting in a terpene-enriched cannabinoid composition with enhanced therapeutic effect for treating anxiety. According to an embodiment, said composition further includes beta-myrcene, and beta-myrcene form less than 5% by weight of the total terpene content, less than 4%, less than 3%, less than 2%, less than 1% or less than 0.5% by weight. Without wishing to be limited by any particular theory, said primary terpenes in said composition may enhance therapeutic effect for treating anxiety via sedating and relieving stress.

According to an embodiment, said composition comprises CBD and optionally THC and/or THCa at CBD to THC and/or THCa weight/weight ratio greater than 1, greater than 2, greater than 3, greater than 4 or greater than 5, and said primary terpene is selected from the group consisting of limonene, linalool, beta-myrcene, and beta-caryophyllene, resulting in a terpene-enriched cannabinoid composition with enhanced therapeutic effect for treating depression. According to an embodiment, said primary terpene is limonene and/or linalool. Without wishing to be limited by any particular theory, said primary terpenes in said composition may enhance therapeutic effect for treating depression via elevating mood and relieving stress.

According to an embodiment, said composition comprises CBD and optionally THC and/or THCa at CBD to THC and/or THCa weight/weight ratio greater than 1, greater than 2, greater than 3, greater than 4 or greater than 5, and said primary terpene is selected from the group consisting of limonene, beta-caryophyllene, caryophyllene oxide, pinene, beta-myrcene, humulene, citronellol, and eucalyptol, resulting in a terpene-enriched cannabinoid composition with enhanced therapeutic effect for treating autism and autism spectrum disorder. According to an embodiment, said primary terpene is selected from the group consisting of beta-caryophyllene, pinene, and beta-myrcene. Without wishing to be limited by any particular theory, said primary terpenes in said composition may enhance therapeutic effect for treating autism and autism spectrum disorder via reducing neuroinflammation and excitotoxicity

According to an embodiment, said composition comprises CBD and/or THC and/or THCa, and said primary terpene is selected from the group consisting of beta-myrcene, linalool, beta-caryophyllene, humulene, eucalyptol and beta-amyrin, resulting in a terpene-enriched cannabinoid composition with enhanced therapeutic effect for treating acute and/or chronic pain including acute and/or chronic pain from_multiple sclerosis, Fibromyalgia, cancer and peripheral neuropathy. According to an embodiment, said primary terpene is selected from the group consisting of beta-myrcene, linalool, and beta-caryophyllene. Without wishing to be limited by any particular theory, said primary terpenes in said composition may enhance therapeutic effect for treating acute and/ or chronic pain via modulating nociceptive thresholds, inhibiting release of pro-inflammatory molecules, and displaying synergistic effects with other systems influencing analgesia.

According to an embodiment, said composition comprises CBD and optionally THC and/or THCa at CBD to THC and/or THCa weight/weight ratio greater than 1, greater than 2, greater than 3, greater than 4 or greater than 5 and said primary terpene is selected from the group consisting of linalool, beta-myrcene, and beta-caryophyllene, resulting in a terpene-enriched cannabinoid composition with enhanced therapeutic effect for treating sleep disorders. According to an embodiment, said primary terpene is linalool and/or beta-myrcene. Without wishing to be limited by any particular theory, said primary terpenes in said composition may enhance therapeutic effect for treating sleep disorders via relieving stress, relaxation and sedation.

According to an embodiment, said terpene-enriched cannabinoid composition of improved therapeutic effect is a humans medication. According to an embodiment, said terpene-enriched cannabinoid composition of improved therapeutic effect is a veterinary medication.

According to an embodiment, the shelf life of said composition is at least 6 months or at least a year. According to an embodiment, primary terpene degradation in said composition is less than 20% per year.

According to an embodiment, further provided is a product comprising tablets, gel capsules, energy drinks, bakery products, medical patches, cigarettes and vaporizer liquids containing said composition.

According to an embodiment, further provided is a commercial product comprising two compositions according to the present invention, which two compositions differ in the content of said primary terpene. According to an embodiment, further provided is a commercial product comprising two compositions according to the present invention, which two compositions comprise different primary terpenes.

According to an embodiment, further provided is a method for producing said composition comprising providing at least one cannabinoid and blending it with a primary terpene.

According to an embodiment, said method includes extracting cannabis plant material to form an extract. According to an embodiment said extracting comprises steam distillation. According to an embodiment, said method further comprises removing terpenes from said extract prior to said blending. According to an embodiment, said extracting comprises contacting with an extractant to form an extract, which extract comprises at least one cannabinoid and said extractant; and optionally removing at least a fraction of said extractant from said extract. According to an embodiment, said extractant comprises at least one of ethanol, a liquefied gas, such as butan, butane or dimethyl-ether, liquefied CO₂, near-critical CO₂ supercritical CO₂ and combinations thereof. According to an embodiment, extracting comprises contacting said cannabis plant material with an extractant to form an extract, and said extractant comprises at least one terpene. According to an embodiment, said extractant comprises said primary terpene.

According to an embodiment, said method further comprises at least partially decarboxylating said cannabinoid. According to an embodiment said decarboxylating is conducted at a temperature greater than 100°C. According to an embodiment said decarboxylating is conducted prior to extracting. According to an embodiment said decarboxylating is conducted on the extract prior to said blending with said primary terpene. According to an embodiment, a fraction of said extract is decarboxylated, and said decarboxylated fraction is blended with another fraction, which was not decarboxylated.

According to an embodiment, said method further comprises extracting a plant material, whereby said primary terpene is produced. According to an embodiment, said plant material is selected from the group consisting of cannabis, lemons, oranges, hops, lavender, pine needles, Echinacea, tea, clover and capsicum.

According to an embodiment, said method comprises synthesizing at least one cannabinoid and blending said synthesized cannabinoid with said primary terpene.

According to an embodiment, said method further comprises blending cannabis plant material with said primary terpene. According to an embodiment, said blending cannabis plant material with said primary terpene comprises spraying said cannabis plant material with said primary terpene, optionally in a solvent. According to an embodiment, said blending cannabis plant material with said primary terpene comprises combining said cannabis plant material with a substrate comprising said primary terpene. According to an embodiment, said substrate is a plant material sprayed with said primary terpene. According to an embodiment, said substrate is a cigarette paper sprayed with said primary terpene.

According to an embodiment, further provided is a method for producing said composition comprising extracting cannabis plant material to form an extract, wherein said extracting forms at least two extract fractions, a cannabinoid-enriched extract and a terpene-enriched extract. As used herein, the term "cannabinoid-enriched extract" refers to an extract wherein cannabinoid to terpenes weight/weight ratio is greater than that in said plant material. As used herein, the term "terpene-enriched extract" refers to an extract wherein cannabinoid to terpenes weight/weight ratio is smaller than that in said plant material. According to an embodiment, said method comprises dividing said cannabinoid-enriched extract into at least two fractions and mixing at least one fraction with at least part of said terpene-enriched extract to form a terpene-enriched composition, wherein terpenes to cannabinoid weight/weight ratio is greater than that ratio in the cannabis plant material. According to an embodiment said ratio is 1.5 times greater than that in the cannabis plant material, 2 times greater, 2.5 times greater, 3 times greater, 4 times greater, 5times greater, 6 times greater, 7 times greater, 8 times greater, or 10 times greater. According to an embodiment, said terpene-enriched composition is further mixed with at least one terpene.

According to an embodiment, further provided is a method for treating a patient comprising administering to said patient said composition. According to an embodiment, said composition is administered in a form selected from the group consisting of cigarettes, vaporizer plant material, vaporizer liquid, extract, tablets, gel capsules, and combinations thereof. According to an embodiment, the daily dose of said composition comprises about 1 milligram cannabinoid to about 300 milligram, about 2 milligram cannabinoid to about 200 milligram or about 3 milligram cannabinoid to about 100 milligram. According to another embodiment, the daily dose of said composition comprises about 0.5 milligram cannabinoid per kilogram body weight to about 30 milligram cannabinoid per kilogram body weight.

According to an embodiment, said method for treating a patient comprises (i) administering to said patient for a first period of time a first terpene-enriched cannabis composition comprising a first cannabinoid at a first cannabinoid amount and a first primary terpene at a first primary terpene amount, followed by (ii) administering to said patient for a second period of time a second terpene-enriched, cannabis composition comprising said first cannabinoid amount and a second primary terpenes at a second primary terpene amount. According to an embodiment, said method further comprises administering to said patient for a third period of time said first terpene-enriched cannabis composition comprising said first cannabinoid amount and a third primary terpene at a third primary terpene amount. According to an embodiment, said third primary terpene is identical to said first primary terpene.

According to another embodiment, said method for treating a patient comprises (i) administering to said patient a first terpene-enriched cannabis composition comprising a first cannabinoid at a first cannabinoid amount and a first primary terpene at a first primary terpene amount and administering to said patient, at least 2 hours later a second terpene-enriched cannabis composition comprising said first cannabinoid amount and a second primary terpenes at a second primary terpene amount. According to another embodiment, said first terpene-enriched cannabis composition is administered for day time and said second terpene-enriched cannabis composition is administered for night time.

According to another embodiment, said method for treating a patient comprises administering to said patient (i) a first terpene-enriched cannabis composition comprising a first cannabinoid at a first cannabinoid amount and a first primary terpene at a first primary terpene amount and (ii) a second terpene-enriched cannabis composition comprising said first cannabinoid and a second primary terpenes at a second primary terpene amount.

According to an embodiment, said method for treating a patient comprises (i) administering to said patient multiple cannabis compositions to find the best working one; (ii) mimicking said best working composition by extracting cannabis plant material to form an extract and blending said extract with suitable terpenes, whereby a mimicking composition is formed and (iii) administering to said patient said mimicking composition. According to an embodiment, said method further comprises removing terpenes from said extract prior to said blending.

### Examples 1- 8

The Table presents examples of compositions as described herein.

| | Form | Cannabinoid | Primary terpene | Content (% by weight) | | Therapeutic effect |
|---|---|---|---|---|---|---|
| | | | | Cannabinoid | Primary terpene | |
| 1 | Cigarette | THCa plus CBD | Limonene | 5-25 plus 5-25 | 1-10 | Anxiety |
| 2 | Oil | CBD | Linalool | 10-40 | 2-20 | Depression |
| 3 | tablets | THCa plus CBD | beta-Caryophyllene | 5-25 plus 5-25 | 1-10 | Autism and autism spectrum disorder |
| 4 | Oil | THC plus CBD | Linalool | 10-40 plus 10-40 | 2-20 | Pain |
| 5 | Extract | CBD | Pinene | 10-40 | 2-20 | Autism and autism spectrum disorder |
| 6 | Oil | THCa plus CBD | beta-Caryophyllene | 10-40 plus 10-40 | 2-20 | Autism and autism spectrum disorder |
| 7 | Cigarette | THC | Beta-myrcene | 5-25 | 1-10 | Pain |
| 8 | Oil | CBD | Linalool | 10-40 | 2-20 | Anxiety |

Thus, the scope of the invention shall include all modifications and variations that may fall within the scope of the attached claims. Other embodiments of the invention will be apparent to those skilled in the art from consideration of the specification and practice of the invention disclosed herein. It is intended that the specification and examples be considered as exemplary only, with a true scope and spirit of the invention being indicated by the following claims.

## Claims

1. A composition in the form of an extract or a product thereof, said composition comprising
(i) at least one cannabinoid;
(ii) a total primary terpene content forming at least 40% by weight of the total amount of terpenes in the composition;
(iii) at least 5% by weight of a non-cannabinoid, non-terpene carrier, wherein said carrier comprises less than 5% cellulose by weight;
(iv) less than 5% by weight glycol; and
(v) less than 5% by weight water;
for use in treating a condition selected from the group consisting of pain, anxiety, depression, a sleep disorder, autism and autism spectrum disorder (ASD) by oral administration,
wherein
a) when said condition is pain, said at least one cannabinoid comprises THC and/or CBD; said primary terpene is selected from the group consisting of beta-myrcene, linalool, beta-caryophyllene, humulene, eucalyptol, beta-amyrin and combinations thereof; and a total terpenes to total cannabinoids weight/weight ratio in said composition is between 0.05:1 and 0.5:1;
b) when said condition is depression, said at least one cannabinoid comprises CBD; said primary terpene is selected from the group consisting of limonene, linalool, beta-myrcene, beta-caryophyllene and combinations thereof; and a total terpenes to total cannabinoids weight/weight ratio in said composition is between 0.1:1 and 0.5:1;
c) when said condition is anxiety, said at least one cannabinoid comprises CBD; said primary terpene is selected from the group consisting of limonene, linalool, beta-caryophyllene and combinations thereof and a total terpenes to total cannabinoids weight/weight ratio in said composition is between 0.1:1 and 0.5:1;
d) when said condition is ASD, said at least one cannabinoid comprises CBD; said primary terpene is selected from the group consisting of limonene, beta-caryophyllene, caryophyllene oxide, pinene, beta- myrcene, humulene, citronellol, eucalyptol and combinations thereof; and a total terpenes to total cannabinoids weight/weight ratio in said composition is between 0.05:1 and 0.5:1; and
e) when said condition is a sleep disorder, said at least one cannabinoid comprises CBD;
said primary terpene is selected from the group consisting of linalool, beta-myrcene, beta-caryophyllene and combinations thereof; and a total terpenes to total cannabinoids weight/weight ratio in said composition is between 0.1:1 and 0.5:1.

2. The composition for use in treating a condition selected from the group consisting of depression, anxiety, autism and ASD according to Claim 1, wherein said at least one cannabinoid further comprises THC and/or THCa at CBD to THC and/or THCa weight/weight ratio greater than 1.

3. The composition for use in treating pain according to Claim 1, wherein said pain comprises acute and/or chronic pain.

4. The composition for use in treating pain according to claim 3, wherein said chronic pain comprises chronic pain from a condition selected from the group consisting of multiple sclerosis, Fibromyalgia, cancer and peripheral neuropathy.

5. The composition for use in treating a condition according to claim 1, wherein at least 50% of said cannabinoid is in decarboxylated form.

6. The composition for use according to any one of Claims 1 to 5, wherein said composition is administered by:
(i) administering to a patient for a first period of time a first terpene-enriched cannabis composition comprising a first cannabinoid at a first cannabinoid amount and a first primary terpene at a first primary terpene amount, followed by
(ii) administering to said patient for a second period of time a second terpene-enriched cannabis composition comprising said first cannabinoid amount and a second primary terpenes at a second primary terpene amount.

## Patentansprüche

1. Zusammensetzung in Form eines Extrakts oder eines Produkts davon, wobei die Zusammensetzung umfasst
(i) mindestens ein Cannabinoid;
(ii) einen insgesamten primären Terpengehalt, der mindestens 40 Gewichts-% der Gesamtmenge an Terpenen in der Zusammensetzung bildet;
(iii) mindestens 5 Gewichts-% eines Nicht-Cannabinoid-, Nicht-Terpen-Trägers, wobei der Träger weniger als 5 Gewichts-% Cellulose umfasst;
(iv) weniger als 5 Gewichts-% Glycol; und
(v) weniger als 5 Gewichts-% Wasser;
zur Verwendung beim Behandeln eines Zustands, der ausgewählt ist aus der Gruppe bestehend aus Schmerz, Angst, Depression, einer Schlafstörung, Autismus und Autismus-Spektrum-Störung (ASD), durch orale Verabreichung,
wobei,
a) wenn es sich bei dem Zustand um Schmerz handelt, das mindestens eine Cannabinoid THC und/oder CBD umfasst; das primäre Terpen ausgewählt ist aus der Gruppe bestehend aus Beta-Myrcen, Linalool, Beta-Caryophyllen, Humulen, Eucalyptol, Beta-Amyrin und Kombinationen davon; und ein Gewicht/Gewicht-Verhältnis von Gesamtterpenen zu Gesamtcannabinoiden in der Zusammensetzung zwischen 0,05:1 und 0,5:1 beträgt;
b) wenn es sich bei dem Zustand um Depression handelt, das mindestens eine Cannabinoid CBD umfasst; das primäre Terpen ausgewählt ist aus der Gruppe bestehend aus Limonen, Linalool, Beta-Myrcen, Beta-Caryophyllen und Kombinationen davon; und ein Gewicht/Gewicht-Verhältnis von Gesamtterpenen zu Gesamtcannabinoiden in der Zusammensetzung zwischen 0,1:1 und 0,5:1 beträgt;
c) wenn es sich bei dem Zustand um Angst handelt, das mindestens eine Cannabinoid CBD umfasst; das primäre Terpen ausgewählt ist aus der Gruppe bestehend aus Limonen, Linalool, Beta-Caryophyllen und Kombinationen davon und ein Gewicht/Gewicht-Verhältnis von Gesamtterpenen zu Gesamtcannabinoiden in der Zusammensetzung zwischen 0,1:1 und 0,5:1 beträgt;
d) wenn es sich bei dem Zustand um ASD handelt, das mindestens eine Cannabinoid CBD umfasst; das primäre Terpen ausgewählt ist aus der Gruppe bestehend aus Limonen, Beta-Caryophyllen, Caryophyllenoxid, Pinen, Beta-Myrcen, Humulen, Citronellol, Eucalyptol und Kombinationen davon; und ein Gewicht/Gewicht-Verhältnis von Gesamtterpenen zu Gesamtcannabinoiden in der Zusammensetzung zwischen 0,05:1 und 0,5:1 beträgt; und
e) wenn es sich bei dem Zustand um Schlafstörung handelt, das mindestens eine Cannabinoid CBD umfasst; das primäre Terpen ausgewählt ist aus der Gruppe bestehend aus Linalool, Beta-Myrcen, Beta-Caryophyllen und Kombinationen davon; und ein Gewicht/Gewicht-Verhältnis von Gesamtterpenen zu Gesamtcannabinoiden in der Zusammensetzung zwischen 0,1:1 und 0,5:1 beträgt.

2. Zusammensetzung zur Verwendung beim Behandeln eines Zustands, der ausgewählt ist aus der Gruppe bestehend aus Depression, Angst, Autismus und ASD nach Anspruch 1, wobei das mindestens eine Cannabinoid ferner THC und/oder THCa bei einem Gewicht/Gewicht-Verhältnis von CBD zu THC und/oder THCa größer als 1 umfasst.

3. Zusammensetzung zur Verwendung beim Behandeln von Schmerz nach Anspruch 1, wobei der Schmerz akuten und/oder chronischen Schmerz umfasst.

4. Zusammensetzung zur Verwendung beim Behandeln von Schmerz nach Anspruch 3, wobei der chronische Schmerz chronischen Schmerz aus einem Zustand umfasst, der ausgewählt ist aus der Gruppe bestehend aus Multipler Sklerose, Fibromyalgie, Krebs und peripherer Neuropathie.

5. Zusammensetzung zur Verwendung beim Behandeln eines Zustands nach Anspruch 1, wobei mindestens 50 % des Cannabinoids in decarboxylierter Form vorliegen.

6. Zusammensetzung zur Verwendung nach einem der Ansprüche 1 bis 5, wobei die Zusammensetzung oral verabreicht wird durch:
(i) Verabreichen, an einen Patienten für einen ersten Zeitraum, einer ersten terpenangereicherten Cannabiszusammensetzung umfassend ein erstes Cannabinoid in einer ersten Cannabinoidmenge und ein erstes primäres Terpen in einer ersten primären Terpenmenge, gefolgt von
(ii) Verabreichen, an den Patienten für einen zweiten Zeitraum, einer zweiten terpenangereicherten Cannabiszusammensetzung umfassend die erste Cannabinoidmenge und ein zweites primäres Terpen in einer zweiten primären Terpenmenge.

## Revendications

1. Composition sous forme d'extrait ou d'un produit de celui-ci, ladite composition comprenant
(i) au moins un cannabinoïde ;
(ii) une teneur totale en terpènes primaires formant au moins 40 % en poids de la quantité totale de terpènes dans la composition ;
(iii) au moins 5 % en poids d'un support non cannabinoïde et non terpénique, ledit support comprenant moins de 5 % de cellulose en poids ;
(iv) moins de 5 % en poids de glycol ; et
(v) moins de 5 % en poids d'eau ;
destinée à être utilisée dans le traitement d'une affection choisie dans le groupe constitué par la douleur, l'anxiété, la dépression, un trouble du sommeil, l'autisme et le trouble du spectre autistique (TSA) par administration orale, dans laquelle
a) lorsque ladite affection est la douleur, ledit au moins un cannabinoïde comprend du THC et/ou du CBD ; ledit terpène primaire est choisi dans le groupe constitué par le bêta-myrcène, le linalol, le bêta-caryophyllène, l'humulène, l'eucalyptol, la bêta-amyrine et leurs combinaisons ; et un rapport poids/poids de terpènes totaux sur cannabinoïdes totaux dans ladite composition est compris entre 0,05:1 et 0,5:1 ;
b) lorsque ladite affection est la dépression, ledit au moins un cannabinoïde comprend du CBD ; ledit terpène primaire est choisi dans le groupe constitué par le limonène, le linalol, le béta-myrcène, le bêta-caryophyllène et leurs combinaisons ; et un rapport poids/poids de terpènes totaux sur cannabinoïdes totaux dans ladite composition est compris entre 0,1:1 et 0,5:1 ;
c) lorsque ladite affection est l'anxiété, ledit au moins un cannabinoïde comprend du CBD ; ledit terpène primaire est choisi dans le groupe constitué par le limonène, le linalol, le béta-caryophyllène et leurs combinaisons et un rapport poids/poids de terpènes totaux sur cannabinoïdes totaux dans ladite composition est compris entre 0,1:1 et 0,5:1 ;
d) lorsque ladite affection est le TSA, ledit au moins un cannabinoïde comprend du CBD ; ledit terpène primaire est choisi dans le groupe constitué par le limonène, le bêta-caryophyllène, l'oxyde de caryophyllène, le pinène, le bêta-myrcène, l'humulène, le citronellol, l'eucalyptol et leurs combinaisons ; et un rapport poids/poids de terpènes totaux sur cannabinoïdes totaux dans ladite composition est compris entre 0,05:1 et 0,5:1 ; et
e) lorsque ladite affection est un trouble du sommeil, ledit au moins un cannabinoïde comprend du CBD ; ledit terpène primaire est choisi dans le groupe constitué par le linalol, le bêta-myrcène, le béta-caryophyllène et leurs combinaisons ; et un rapport poids/poids de terpènes totaux sur cannabinoïdes totaux dans ladite composition est compris entre 0,1:1 et 0,5:1.

2. Composition destinée à être utilisée dans le traitement d'une affection choisie dans le groupe constitué par la dépression, l'anxiété, l'autisme et le TSA selon la revendication 1, dans laquelle ledit au moins un cannabinoïde comprend en outre du THC et/ou du THCa à un rapport poids/poids de CBD sur THC et/ou THCa supérieur à 1.

3. Composition destinée à être utilisée dans le traitement de la douleur selon la revendication 1, ladite douleur comprenant une douleur aiguë et/ou chronique.

4. Composition destinée à être utilisée dans le traitement de la douleur selon la revendication 3, ladite douleur chronique comprenant une douleur chronique due à une affection choisie dans le groupe constitué par la sclérose en plaques, la fibromyalgie, le cancer et la neuropathie périphérique.

5. Composition destinée à être utilisée dans le traitement d'une affection selon la revendication 1, dans laquelle au moins 50 % dudit cannabinoïde est sous forme décarboxylée.

6. Composition destinée à être utilisée selon l'une quelconque des revendications 1 à 5, ladite composition étant administrée par :
(i) l'administration à un patient, pendant une première période, d'une première composition de cannabis enrichie en terpènes comprenant un premier cannabinoïde à une première quantité de cannabinoïde et un premier terpène primaire à une première quantité de terpène primaire, suivie de
(ii) l'administration audit patient, pendant une seconde période, d'une seconde composition de cannabis enrichie en terpènes comprenant ladite première quantité de cannabinoïde et des seconds terpènes primaires à une seconde quantité de terpènes primaires.
